(19) **Europäisches Patentamt / European Patent Office / Office européen des brevets**

(11) **EP 2 062 553 B1**

(12) **EUROPEAN PATENT SPECIFICATION**

(45) Date of publication and mention
of the grant of the patent:
**25.08.2010 Bulletin 2010/34**

(51) Int Cl.:
*A61F 2/16* (2006.01)          *A61F 9/00* (2006.01)
*G02C 7/02* (2006.01)

(21) Application number: **09154536.8**

(22) Date of filing: **04.04.2006**

(54) **Optimal iol shape factors for ophthalmic lenses**

Optimale Formfaktoren für ophthalmische Linsen

Facteurs de forme pour de lentilles ophthalmiques

(84) Designated Contracting States:
**AT BE BG CH CY CZ DE DK EE ES FI FR GB GR HU IE IS IT LI LT LU LV MC NL PL PT RO SE SI SK TR**

(30) Priority: **05.04.2005 US 668520 P**

(43) Date of publication of application:
**27.05.2009 Bulletin 2009/22**

(62) Document number(s) of the earlier application(s) in accordance with Art. 76 EPC:
**06749285.0 / 1 753 372**

(73) Proprietor: **Alcon, Inc.**
**6331 Hünenberg (CH)**

(72) Inventors:
• **Hong, Xin**
**Fort Worth, TX 76135-9653 (US)**
• **Van Noy, Stephen J.**
**Fort Worth, TX 76132 (US)**

• **Xie, Jihong**
**Jacksonville**
**Florida 32258 (US)**
• **Stanley, Dan**
**Midlothian, TX 76065 (US)**
• **Karakelle, Mutlu**
**Fort Worth, TX 76132 (US)**
• **Simpson, Michael J.**
**Arlington, TX 76102 (US)**
• **Zhang, Xiaoxiao**
**Fort Worth, TX 76132 (US)**

(74) Representative: **Hanna, Peter William Derek et al**
**Hanna Moore & Curley**
**13 Lower Lad Lane**
**Dublin 2 (IE)**

(56) References cited:
**WO-A-2005/098518          JP-A- 4 126 144**
**US-A- 4 504 982          US-A- 5 050 981**
**US-A1- 2003 093 149**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Background

The present invention relates generally to ophthalmic lenses, and more particularly, to intraocular lenses (IOLs) having optimal shape factors.

Intraocular lenses are routinely implanted in patients' eyes during cataract surgery to replace the clouded natural lens. The post-operative performance of such IOLs, however, can be degraded due to a variety of factors. For example, aberrations introduced as a result of misalignment of the implanted IOL relative to the cornea, and/or the inherent aberrations of the eye, can adversely affect the lens's optical performance.

Patent Abstracts of Japan vol. 16, no. 387 (C-0975) 18th August 1992 and JP 04 126144 A (NIDEK Co. Ltd.) describe an ophthalmic lenses exhibiting shape factors of between 0.5 and 1.69, between -0.5 and 1.5 and between -0.4 and 0.6.

US-A-5,050,981 (Roffman, Jeffrey H.) describes an ophthalmic lens having an aspheric base profile and being made of a polymeric material.

US 2003/093149-A1 (Alan N. Glazier) describes a multifocal intraocular lens having an anterior surface and a posterior surface at least one of which is aspheric, exemplified by a lens having an anterior radius of curvature of 10.2 mm and a posterior radius of curvature of -6.00 mm.

Accordingly, there is a need for improved IOLs that can provide a more robust optical performance.

Summary

The present invention provides an ophthalmic lens in accordance with the claims which follow. In one aspect, the present invention provides an ophthalmic lens (e.g., an intraocular lens) having an optic with an anterior surface and a posterior surface. The optic exhibits a shape factor in a range of about -0.5 to about 4. In a related aspect, the shape factor of the optic lies in a range of about 0 to about 2. The above shape factors give rise to a plurality of different lens shapes, such as, biconvex, plano-convex, plano-concave and convex-concave.

The optic is formed of a biocompatible polymeric material. By way of example, the optic can be formed of a soft acrylic polymeric material. Other examples of suitable materials include, without limitation, hydrogel and silicone materials.

At least one surface of the optic is characterized by an aspheric base profile (i.e., a base profile that exhibits deviations from sphericity). The base profile can be characterized by a conic constant in a range of about -73 to about -27.

In a related aspect, the aspheric profile of the lens surface can be defined in accordance with the following relation:

$$z = \frac{cr^2}{1 + \sqrt{1 - (1+k)c^2 r^2}}$$

wherein,

  $c$ denotes the curvature of the surface at its apex (at its intersection with the optical axis),
  $r$ denotes the radial distance from the optical axis, and
  $k$ denotes the conic constant,

wherein

  $c$ can be, e.g., in a range of about 0.0152 mm$^{-1}$ to about 0.0659 mm$^{-1}$,
  $r$ can be, e.g., in a range of about 0 to about 5, and
  $k$ can be, e.g., in a range of about -1162 to about -19 (e.g., in a range of about-73 to about -27).

In a related aspect, the optic of the above lens can have a shape factor in a range of about 0 to about 2.

In some embodiments in which one or more surfaces of the ophthalmic lens exhibit asphericity, the shape factor of the lens (e.g., an IOL) can be selected as a function of that asphericity so as to optimize the lens's optical performance. The invention provides an ophthalmic lens having an optic with an anterior surface and a posterior surface, where at least one of the surfaces exhibits an ashperical profile characterized by a conic constant in a range of about -73 to about -27. The optic exhibits a shape factor in a range of about -0.5 to about 4.

In a related aspect, the ophthalmic lens including at least one aspherical surface characterized by a conic constant in a range of about -73 to about -27 has an optic with a shape factor in a range of about 0 to about 2.

In other aspects, an intraocular lens adapted for implantation in an eye having a corneal radius equal to or less than about 7.1 mm is disclosed, which includes an optic having an anterior surface and a posterior surface. The optic exhibits a shape factor in a range of about -0.5 to about 4. In a related aspect, the optic exhibits a shape factor in a range of about +0.5 to about 4, or in a range of about 1 to about 3.

In another aspect, the invention provides an intraocular lens adapted for implantation in an eye having a corneal radius in a range of about 7.1mm to about 8.6 mm, which includes an optic having an anterior surface and a posterior surface. The optic exhibits a shape factor in a range of about 0 to about 3. In a related aspect, the optic exhibits a shape factor in a range of about +0.5 to about 3, or in a range of about 1 to about 2.

In another aspect, an intraocular lens adapted for implantation in an eye having a corneal radius equal to or greater than about 8.6 is disclosed, which includes an optic having an anterior surface and a posterior surface. The optic exhibits a shape factor in a range of about 0.5 to about 2. In a related aspect, the optic exhibits a shape factor in a range of about 1 to about 2.

In another aspect, the invention provides an intraocular lens adapted for implantation in an eye having an axial length equal to or less than about 22 mm, which includes an optic having an anterior surface and a posterior surface. The optic can have ' a shape factor in a range of about 0 to about 2, or in a range of about 0.5 to about 2.

In other aspects, the invention can be used in methods for selecting an ophthalmic lens for implantation in a patient's eye based on one or more ocular biometric parameters of the patient. For example, a method of correcting vision is disclosed that includes selecting an IOL, which comprises an optic exhibiting a shape factor in a range of about - 0.5 to about 4 (or in a range of about +0.5 to about 4), for implantation in an eye having a corneal radius that is equal to or less than about 7.1 mm.

In another aspect, the invention can be used in a method of correcting vision that includes selecting an IOL, which comprises an optic exhibiting a shape factor in a range of about 0 to about 3 (or in a range of about 0.5 to about 3), for implantation in an eye having a corneal radius in a range of about 7.1 mm to about 8.6 mm

In yet another aspect, the invention can be used in a method of correcting vision that includes selecting an IOL, which comprises an optic exhibiting a shape factor in a range of about 0.5 to about 2, for implantation in an eye having a corneal radius that is equal to or greater than about 8.6 mm.

In another aspect, the invention can be used in a method of corrected vision that includes selecting an IOL, which comprises an optic exhibiting a shape factor in a range of about 0 to about 2 (or in a range of about 0.5 to about 2), for implantation in an eye having an axial length equal to or less than about 22 mm.

In another aspect, the invention can be used in a method of designing an ophthalmic lens that includes defining an error function, which is indicative of variability in performance of a lens in a patient population, based on estimated variability in one or more biometric parameters associated with that population, and selecting a shape factor for the lens that reduces the error function relative to a reference value. In a related aspect, the error function can further include an estimated error in optical power correction provided by the lens and/or an estimated aberration error.

In a related aspect, the error function (*RxError*) can be defined in accordance with the following relation:

$$RxError = \sqrt{\Delta Biometric^2 + \Delta IOLPower^2 + \Delta Aberration^2}$$

wherein,

$\Delta Biometric$ denotes variability due to biometric data errors,
$\Delta IOLPower$ denotes variability due to optical power correction errors, and
$\Delta Aberration$ denotes variability due to aberration contributions.

In another aspect, the $\Delta Biometric$ can be defined in accordance with the following relation:

$$\Delta Biometric = \sqrt{\Delta k^2 + \Delta AL^2 + \Delta ACD^2}$$

wherein,

$\Delta k$ denotes error in keratometric measurements,
$\Delta AL$ denotes error in axial length measurements, and

$\Delta ACD$ denotes error in anterior chamber depth measurements.

In another aspect, the $\Delta Aberration$ can be defined in accordance with the following relation:

$$\Delta Aberration = \sqrt{\Delta Astig^2 + \Delta SA^2 + \Delta Other^2}$$

wherein,

$\Delta Astig$ represents variability due to astigmatic aberration,
$\Delta SA$ represents variability due to spherical aberration, and
$\Delta Other$ represents variability due to other aberrations.

In a further aspect, the $\Delta IOLPower$ can be defined in accordance with the following relation:

$$\Delta IOLPower = \sqrt{\Delta IOLStep^2 + \Delta IOLTol^2 + \Delta ELP^2}$$

wherein,

$\Delta IOLStep$ represents variability caused by difference between a power correction provided by the lens and a power correction needed by a patient,
$\Delta IOLTol$ represents manufacturing power tolerance, and
$\Delta ELP$ represents variability in a shift of the lens effective position within the eye.

Further understanding of the invention can be obtained by reference to the following detailed description, in conjunction with the associated drawings, which are discussed briefly below.

**Brief Description of the Drawings**

FIGURE 1 is a schematic side view of an example IOL having spherical surfaces.

FIGURE 2 presents simulated magnitude of different aberration types (spherical, defocus, coma and astigmatic aberrations) exhibited by an IOL as a function of its shape factor for a 1,5 mm decentration,

FIGURE 3 presents simulation results for aberrations exhibited by an IOL due to tilt as a function of the IOL's shape factor,

FIGURE 4A presents graphically calculated spherical aberration exhibited by a model eye characterized by an average anterior chamber depth in which an IOL is incorporated, as a function of the IOL's shape factor,

FIGURE 4B presents graphically calculated MTFs at 50 lp/mm and 100 lp/mm for a model eye characterized by an average anterior chamber depth in which an IOL is incorporated, as a function of the IOL's shape factor,

FIGURE 5A depicts simulated MTFs at 50 lp/mm and 100 lp/mm for a model eye characterized by a small anterior chamber depth in which an IOL is incorporated, as a function of the IOL's shape factor,

FIGURE 5B depicts simulated spherical aberration exhibited by a model eye characterized by a small anterior chamber depth in which an IOL is incorporated, as a function of the IOL's shape factor,

FIGURE 6A depicts simulated spherical aberration exhibited by a model eye characterized by a large anterior chamber depth in which an IOL is incorporated, as a function of the IOL's shape factor,

FIGURE 6B depicts simulated MTFs at 50 lp/mm and 100 lp/mm for a model eye characterized by a large anterior chamber depth in which an IOL is incorporated, as a function of the IOL's shape factor,

FIGURE 7A depicts graphically simulated spherical aberrations exhibited by a plurality of model eyes having different corneal asphericities in which an IOL is incorporated, as a function of the IOL's shape factor,

FIGURE 7B depicts graphically simulated MTF as 50 lp/mm obtained for model eyes having different corneal asphericities in which an IOL is incorporated, as a function of the IOL's shape factor,

FIGURE 7C depicts graphically simulated MTF at 100 lp/mm obtained for model eyes having different corneal asphericities in which an IOL is incorporated, as a function of the IOL's shape factor,

FIGURE 8A depicts simulated spherical aberration exhibited by two model eyes characterized by different corneal radii as a function of the shape factor of an IOL incorporated in the models,

FIGURE 8B depicts simulated MTF at 50 lp/mm exhibited by two model eyes characterized by different corneal radii as a function of the shape factor of an IOL incorporated in the models,

FIGURE 8C depicts simulated MTF at 100 lp/mm exhibited by two model eyes characterized by different corneal radii as a function of the shape factor of an IOL incorporated in the models,

FIGURE 9A depicts simulated spherical aberration exhibited by a plurality of model eyes having different axial lengths as a function of the shape factor of an IOL incorporated in the models,

FIGURE 9B depicts simulated MTFs at 50 lp/mm exhibited by a plurality of model eyes having different axial lengths as a function of the shape factor of an IOL incorporated in the models,

FIGURE 9C depicts simulated MTFs at 100 lp/mm exhibited by a plurality of model eyes having different axial lengths as a function of the shape factor of an IOL incorporated in the models,

FIGURE 10 is a schematic side view of a lens according to one embodiment of the invention having an aspheric anterior surface,

FIGURE 11 presents a plurality of graphs depicting the sag of an aspheric surface of two lenses in accordance with the teachings of the invention having different shape factors, and

FIGURE 12 graphically presents Monte Carlo simulation results for optical performance of a plurality of IOLs as a function of manufacturing tolerances.

**Detailed Description of the Preferred Embodiments**

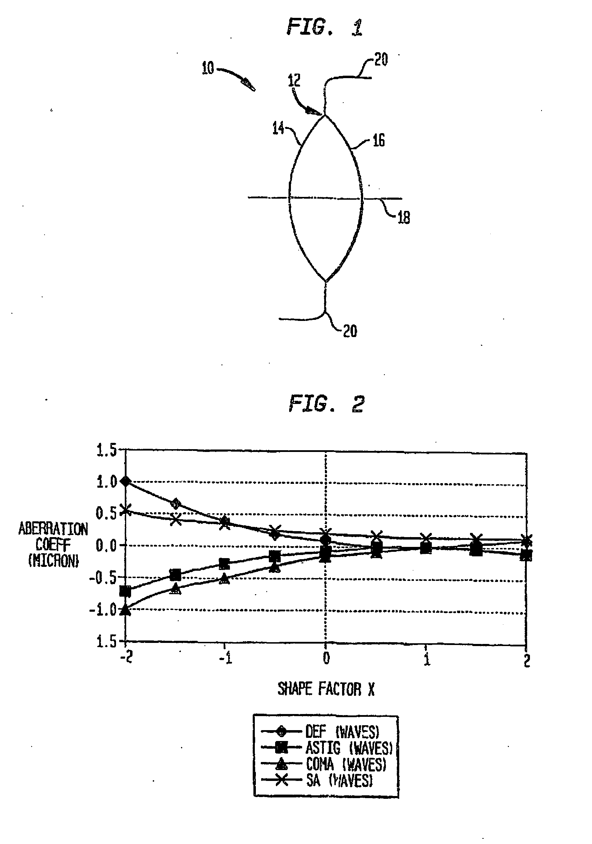

FIGURE 1 schematically depicts an IOL 10 having an optic 12 that includes an anterior surface 14 and a posterior surface 16. In this embodiment, the anterior and posterior surfaces 14 and 16 are symmetrically disposed about an optical axis 18, though in other embodiments one or both of those surfaces can exhibit a degree of asymmetry relative to the optical axis. The exemplary IOL 10 further includes radially extending fixation members or haptics 20 that facilitate its placement in the eye. In this embodiment, the optic is formed of a soft acrylic polymer, commonly known as Acrysof, though in other embodiments, it can be formed of other biocompatible materials, such as silicone or hydrogel The lens 10 provides a refractive optical power in a range of about 6 to about 34 Diopters (D), and preferably in a range of about 16 D to about 25 D.

In this exemplary embodiment, the lens 10 has a shape factor in a range of about 0 to about 2. More generally, in many embodiments, the shape factor of the lens 10 can range from about -0.5 to about 4. As known in the art, the shape factor of the lens 10 can be defined in accordance with the following relation:

$$\text{Shape Factor (X)} = \frac{C_1 + C_2}{C_1 - C_2} \qquad \text{Eq. (1)}$$

wherein $C_1$ and $C_2$ denote, respectively, the curvatures of the anterior and posterior surfaces.

The shape factor of the IOL 10 can affect the aberrations (e.g., spherical and/or astigmatic aberrations) that the lens can introduce as a result of its tilt and decentration, e.g., when implanted in the subject's eye or in a model eye. As discussed in more detail below, aberrations caused by a plurality of IOLs with different shape factors were theoretically studied as a function of tilt and decentration by utilizing a model eye. Those studies indicate that IOLs having a shape factor in a range of about 0 to about 2 introduce much reduced aberrations as a result of tilt and decentration.

More particularly, to study the effects of an IOL's shape factor on aberrations induced by its tilt and decentration, a hypothetical eye model having optical properties (e.g., corneal shape) similar to those of an average human eye was employed. The radii of optical surfaces and the separations between optical components were chosen to correspond to mean values of those parameters for the human population. The refractive indices of the optical components were chosen to provide selected refractive power and chromatic aberrations. Further, the anterior corneal surface of the model was selected to have an ashperical shape. An IOL under study replaced the natural lens in the model. Table 1 below lists the various design parameters of the model eye:

**Table 1**

| Surface | Type | Radius (mm) | Thickness (mm) | Class | Diameter (mm) | Conic Constant |
|---|---|---|---|---|---|---|
| OBJ | Standard | Infinity | Infinity | | 0.000 | 0.000 |
| 1 | Standard | Infinity | 10,000 | | 5.000 | 0.000 |
| 2 | Standard | 7.720 | 0.550 | Cornea | 14.800 | -0.260 |
| 3 | Standard | 6.500 | 3.050 | Aqueous | 12.000 | 0.000 |
| STO | Standard | Infinity | 0.000 | Aqueous | 10.000 | 0.000 |
| 5 | Standard | 10.200 | 4.000 | Lens | 11.200 | -3.132 |
| 6 | Standard | -6.000 | 16.179 | Vitreous | 11.200 | -1.000 |
| IMA | Standard | -12.000 | 24.000 | | 0.000 | |

An optical design software marketed as Zemax® (version March 4, 2003, Zemax Development Corporation, San Diego, CA) was utilized for the simulations of the optical properties of the model eye. A merit function was defined based on the root-mean-square (RMS) wavefront aberration, that is, the RMS wavefront deviation of an optical system from a plane wave. In general, the larger the RMS wavefront error, the poorer is the performance of the optical system An optical system with an RMS wavefront error that is less than about 0.071 waves is typically considered as exhibiting a diffraction-limited optical performance.

The effects of misalignment (tilt and/or decentration) of an IOL on its optical performance for a number of different shape factors was simulated by placing the IOLs in the above model eye and utilizing the Zemax® software. For these simulations, the IOL was assumed to have spherical surfaces so as to investigate the effects of the shape factor alone (as opposed to that of the combined shape factor and asphericity). To simulate the scotopic viewing conditions for old patients, a 5 mm entrance pupil was chosen. The following misalignment conditions were considered: 1.5 mm IOL decentration and a 10-degree IOL tilt. These two conditions represent the extreme cases of IOL misalignments.

FIGURE 2 presents the simulated magnitude of different aberration types (spherical aberration, defocus, coma and astigmatism) as a function of the shape factor for 1.5 mm decentration of the IOL. These simulations indicate that IOLs with a shape factor in a range of about 0 to about 2 exhibit much lower aberrations as a result of the decentration. For example, an IOL with a shape factor of about 1 introduces a defocus aberration of 0.07 D compared to a defocus aberration of 0.32 D introduced by an IOL having a shape factor of -1.

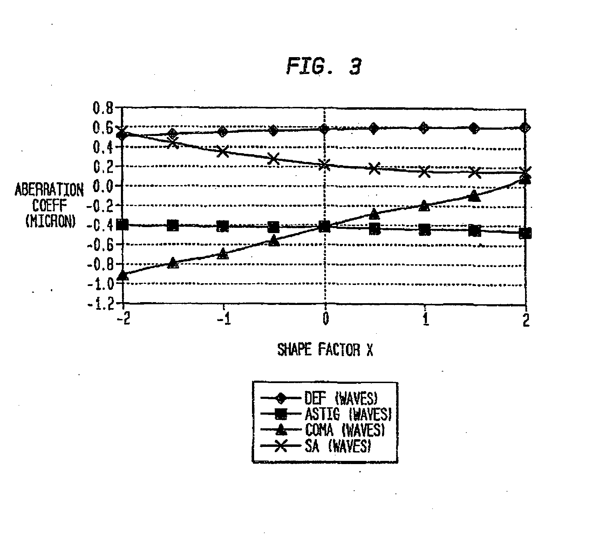

FIGURE 3 presents the simulation results for aberrations introduced as a result of the IOL's tilt. These results indicate that the defocus and astigmatic aberrations are not significantly influenced by the IOL's shape factor while the coma and spherical aberrations exhibit even stronger dependence on the shape factor than their dependence in case of the IOL's decentration. Again, the IOLs with shape factors in a range of about 0 to 2 exhibit a stable performance.

In other aspects, it has been discovered that certain biometric parameters of the eye (e.g., corneal radius and axial length) can be considered while selecting the shape factor of an IOL for implantation in the eye to provide enhanced performance of the lens. As discussed in more detail below, in some embodiments, optimal IOL shape factors are provided for different eye populations, e.g., average human eye (eyes with average values for certain biometric parameters), and other populations characterized by extreme values for those parameters.

The biometric parameters of the above eye model were varied to simulate the performance of a plurality of IOLs having different shape factors for different eyes. For an average human eye, a corneal radius (r) of 7.72 mm, a corneal asphericity (Q) of - 0.26, an anterior chamber depth (ACD) of 4.9 mm, and an axial length (AL) of 24.4 mm were assumed. To investigate human eyes with extreme large or small biometric values, the anterior chamber depth was varied from 4.3 mm to 5.5 mm, the corneal asphericity was varied from -0.50 to 0, the corneal radius was varied from 7.10 mm to 8.60 mm, and the axial length was varied from 22.0 mm to 26.0 mm These ranges are sufficiently broad to cover the values exhibited by the majority of the population. The optical performance of the IOLs was evaluated based on two criteria: calculated wave aberration and modulation transfer function (MTF). As known to those having ordinary skill in the art, the MTF provides a quantitative measure of image contrast exhibited by an optical system, e.g., a system formed of an IOL and the cornea. More specifically, the MTF of an imaging system can be defined as a ratio of a contrast associated with an image of an object formed by the optical system relative to a contrast associated with the object.

Table 2 below presents the simulation results of the optical performance of IOLs having shape factors in a range of about -2 to about 4 for an eye having an average anterior chamber depth (ACD) of 4.9 mm, a corneal radius of 7.72 mm, a corneal asphericity of -0.26, and an axial length (AL) of 24.4 mm, at a pupil size of 5 mm.

**Table 2.**

| Shape Factor (X) | Spherical Aberration (SA) | MTF at 50 lp/mm | MTF at 100 lp/mm |
|---|---|---|---|
| -2 | 0.478 | 0.037 | 0.095 |
| -1.5 | 0.386 | 0.117 | 0.051 |
| -1 | 0.307 | 0.212 | 0.011 |
| -0.5 | 0.244 | 0.331 | 0.016 |
| 0 | 0.195 | 0.455 | 0.128 |
| 0.5 | 0.162 | 0.555 | 0.250 |
| 1 | 0.142 | 0.615 | 0.334 |
| 1.5 | 0.134 | 0.637 | 0.366 |
| 2 | 0.138 | 0.625 | 0.348 |
| 3 | 0.174 | 0.516 | 0.199 |
| 4 | 0.239 | 0.340 | 0.021 |

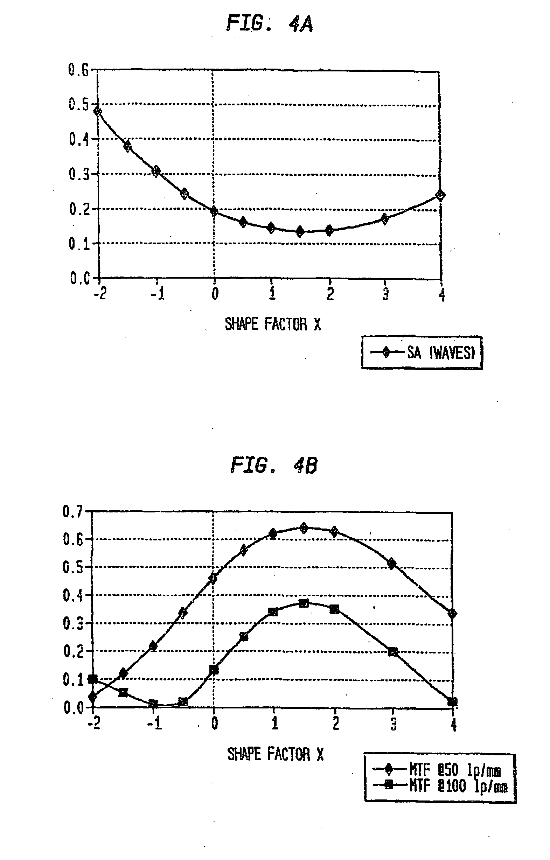

For graphical presentation of the information in Table 2, FIGURES 4A and 4B provide, respectively, the calculated spherical aberration and MTF presented in Table 1 as a function of IOL's shape factor.

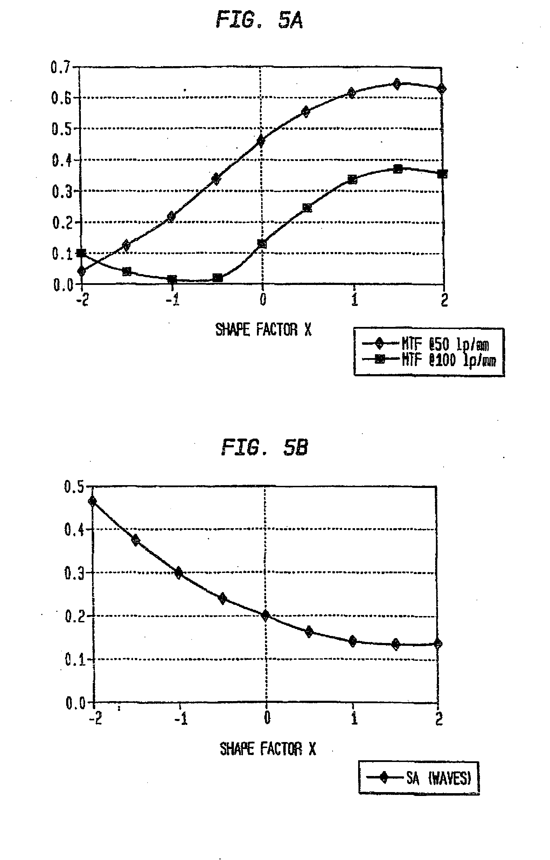

Table 3 below presents the simulation results for the optical performance of a plurality of IOLs having shape factors in the above range of -2 to 4 at a pupil size of 5 mm for an eye having a small anterior chamber depth (ACD) of 4.3 mm, but the same corneal radius (7.72 mm) and asphericity (-0.26) as well as axial length (24.4 mm) as that employed in the previous simulation. FIGURES 5A and 5B graphically depict, respectively, the calculated spherical aberration (SA) and the MTF presented in Table 3 as a function of the IOL's shape factor.

**Table 3.**

| Shape Factor (X) | Sph. Aberration (waves) | MTF at 50 lp/mm | MTF at 100 lp/mm |
|---|---|---|---|
| -2 | 0.461 | 0.047 | 0.095 |
| -1.5 | 0.374 | 0.125 | 0.042 |
| -1 | 0.300 | 0.219 | 0.014 |
| -0.5 | 0.240 | 0.337 | 0.021 |
| 0 | 0.194 | 0.457 | 0.130 |
| 0.5 | 0.161 | 0.553 | 0.249 |
| 1 | 0.141 | 0.613 | 0.331 |
| 1.5 | 0.133 | 0.636 | 0.365 |
| 2 | 0.136 | 0.627 | 0.353 |

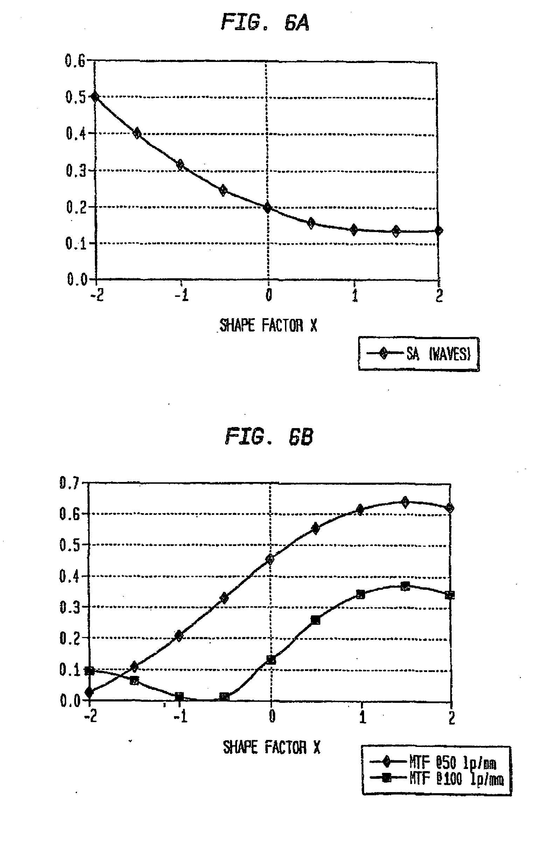

Table 4 below presents the simulation results for the optical performance of a plurality of IOLs having shape factors in the above range of -2 to 4 at a pupil size of 5 mm for an eye having a large anterior chamber depth (ACD) of 5.5 mm, a corneal radius of 7.72 mm, a corneal asphericity of -0.26 and an axial length of 24.4 mm. Further, FIGURES 6A and 6B graphically depict, respectively, the calculated spherical aberration (SA) and the MTF presented in Table 4 as a function of the IOL's shape factor.

**Table 4.**

| Shape Factor (X) | Sph. Aberration (waves) | MTF at 50 lp/mm | MTF at 100 lp/mm |
|---|---|---|---|
| -2 | 0.498 | 0.026 | 0.093 |
| -1.5 | 0.399 | 0.108 | 0.059 |
| -1 | 0.316 | 0.204 | 0.008 |
| -0.5 | 0.249 | 0.325 | 0.011 |
| 0 | 0.198 | 0.454 | 0.125 |
| 0.5 | 0.162 | 0.556 | 0.251 |
| 1 | 0.142 | 0.617 | 0.336 |
| 1.5 | 0.135 | 0.637 | 0.365 |
| 2 | 0.140 | 0.622 | 0.342 |

These simulations indicate that IOLs with shape factors in a range of about -0.5 to about 4, and particularly those having shape factors in a range of about 0 to about 2, provide enhanced optical performance. The simulations, however, show that anterior chamber depth does not significantly affect the performance of an IOL.

Although in the afore-mentioned simulations the spherical aberrations were considered, if the IOL is misaligned relative to the cornea, other aberrations (e.g., defocus, astigmatism and coma) can also be present. The simulations of these aberrations for average, small and large ACD confirm that the aberrations can be minimized by utilizing shape factors in a range about 0 to about 2.

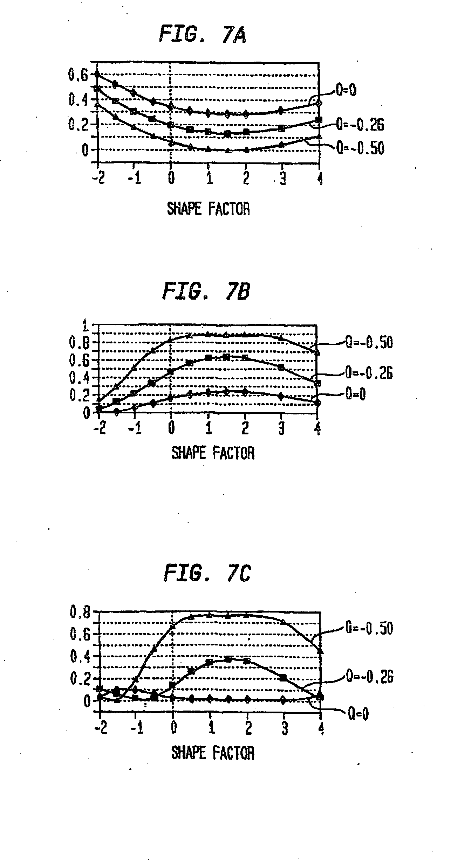

The impact of corneal asphericity (Q) on optimal IOL shape factor was also investigated by utilizing the afore-mentioned eye model and calculating spherical aberration and MTF for Q = 0 (spherical), Q = -0.26 and Q = -0.50. The more negative the Q value, the flatter is the peripheral portion of the cornea. Q = -0.26 corresponds to the asphericity of the normal human cornea while Q = -0.50 corresponds to the asphericity of an extremely flat cornea. Table 5 below lists the results of these simulations, with FIGURES 7A, 7B and 7C graphically depicting, respectively, the simulated spherical aberration, the MTF at 50 lp/mm and the MTF at 100 lp/mm as a function of the IOL's shape factor.

**Table 5**

| | SA (micron) | | | MTF@50lp/mm | | | MTF@100lp/mm | | |
|---|---|---|---|---|---|---|---|---|---|
| X | Q = 0 | Q = -0.26 | Q =-0.50 | Q = 0 | Q = -0.26 | Q = -50 | Q = 0 | Q = -0.26 | Q = -0.50 |
| -2 | 0.609 | 0.478 | 0.364 | 0.000 | 0.037 | 0.143 | 0.036 | 0.095 | 0.027 |
| -1.5 | 0.524 | 0.386 | 0.264 | 0.010 | 0.117 | 0.292 | 0.084 | 0.051 | 0.007 |
| -1 | 0.451 | 0.307 | 0.180 | 0.058 | 0.212 | 0.503 | 0.091 | 0.011 | 0.182 |
| -0.5 | 0.392 | 0.244 | 0.112 | 0.111 | 0.331 | 0.702 | 0.057 | 0.016 | 0.463 |
| 0 | 0.347 | 0.195 | 0.061 | 0.159 | 0.455 | 0.822 | 0.016 | 0.128 | 0.661 |
| 0.5 | 0.3l5 | 0.162 | 0.025 | 0.200 | 0.555 | 0.869 | 0.007 | 0.250 | 0.742 |
| 1 | 0.295 | 0.142 | 0.005 | 0.230 | 0.615 | 0.879 | 0.012 | 0.334 | 0.759 |
| 1.5 | 0.288 | 0.134 | 0.002 | 0.243 | 0.637 | 0.879 | 0.012 | 0.366 | 0.759 |
| 2 | 0.29 | 0.138 | 0.003 | 0.238 | 0.625 | 0.879 | 0.013 | 0.348 | 0.759 |
| 3 | 0.321 | 0.174 | 0.045 | 0.189 | 0.516 | 0.848 | 0.004 | 0.199 | 0.704 |
| 4 | 0.378 | 0.239 | 0.117 | 0.120 | 0.340 | 0.688 | 0.046 | 0.021 | 0.443 |

The spherical aberration exhibited by a spherical cornea (Q=0) is significantly larger than those exhibited by the aspherical corneas (Q = -0.26 and Q = -0.50), as expected. As a result, the MTFs associated with Q = 0 are lower than those for Q =-0.26 and Q = -0.50. However, for each of the three cases, the above simulations indicate that an optimal IOL shape factor lies in a range of about -0.5 to about 4, and preferably in a range of about 0 to about 2.

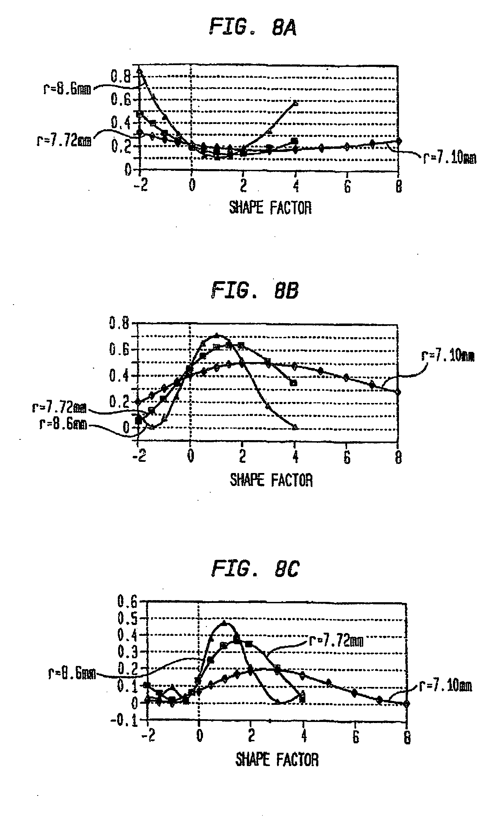

In another set of simulations, the effect of corneal radius on optimal shape factor was investigated. Table 6 below presents the simulation results corresponding to spherical aberration as well as MTFs at 50 lp/mm and 100 lp/mm obtained for a plurality of IOLs having shape factors in a range of about -2 to about 8 by utilizing the aforementioned eye model and varying the corneal radius. More specifically, the ACD, Q and AL were fixed, respectively, at 4.9 mm, -0.26, and 24.4 mm while the corneal radius was varied. FIGURE 8A, 8B and 8C graphically depict, respectively, variations of the spherical aberration, the MTF at 50 lp/mm and the MTF at 100 lp/mm in these simulations as a function of tha IOL's shape factor for two different radii.

**Table 6**

| r | SA (waves) | | | MTF@50lp/mm | | | MTF@100lp/mm | | |
|---|---|---|---|---|---|---|---|---|---|
| X | r=7.10 | r=7.72 | r=8.60 | r=7.10 | r=7.72 | r=8.60 | r=7.10 | r=7.72 | r=8.60 |
| | mm | mm | mm | mm | mm | mm | mm | mm | mm |
| -2 | 0.312 | 0.478 | 0.856 | 0.196 | 0.037 | 0.086 | 0.010 | 0.095 | 0.031 |
| -1.5 | 0.282 | 0.386 | 0.635 | 0.245 | 0.117 | 0.00 | 0.015 | 0.051 | 0.032 |
| -1 | 0.255 | 0.307 | 0.447 | 0.297 | 0.212 | 0.07 | 0.002 | 0.011 | 0.086 |
| -0.5 | 0.233 | 0.244 | 0.300 | 0.347 | 0.331 | 0.234 | 0.029 | 0.016 | 0.011 |
| 0 | 0.215 | 0.195 | 0.195 | 0.393 | 0.455 | 0.468 | 0.067 | 0.128 | 0.139 |
| 0.5 | 0.201 | 0.162 | 0.133 | 0.432 | 0.555 | 0.65 | 0.105 | 0.250 | 0.382 |
| 1 | 0.190 | 0.142 | 0.111 | 0.463 | 0.615 | 0.711 | 0.139 | 0.334 | 0.476 |
| 1.5 | 0.182 | 0.134 | 0.127 | 0.485 | 0.637 | 0.667 | 0.165 | 0.366 | 0.408 |
| 2 | 0.177 | 0.138 | 0.174 | 0.499 | 0.625 | 0.528 | 0.182 | 0.348 | 0.210 |
| 3 | 0.175 | 0.174 | 0.344 | 0.503 | 0.516 | 0.173 | 0.188 | 0.199 | 0.008 |
| 4 | 0.182 | 0.239 | 0.579 | 0.483 | 0.340 | 0.008 | 0.163 | 0.021 | 0.062 |
| 5 | 0.195 | - | - | 0.444 | - | - | 0.118 | - | - |
| 6 | 0.213 | - | - | 0.394 | - | - | 0.067 | - | - |
| 7 | 0.234 | - | - | 0.339 | - | - | 0.022 | - | - |
| 8 | 0.258 | - | - | 0.285 | - | - | 0.007 | - | - |

These simulations indicate that for a very steep cornea (e.g., a corneal radius of 7.1 mm), the IOL's shape factor has a relatively small impact on the spherical aberration and the MTF. For example, in such a case, for shape factors in a wide range of about -1 to about 8, good optical performance is observed, though shape factors in a range of about 0.5 to about 4 are preferred. However, for a cornea having a large radius, e.g., a radius larger than about 8.6 mm, an optimal range of about 0 to about 2 (e.g., about 0.5 to about 2) for the IOL's shape factor is observed. The peak of the IOL's optical performance as a function of the shape factor also shifts as the corneal radius varies from a small value to a large one. For example, the simulations indicate a peak performance at a shape factor of about 3 for a cornea with a radius of about 7.1 mm and at a shape factor of about 1 for a cornea with a radius of about 8.6 mm.

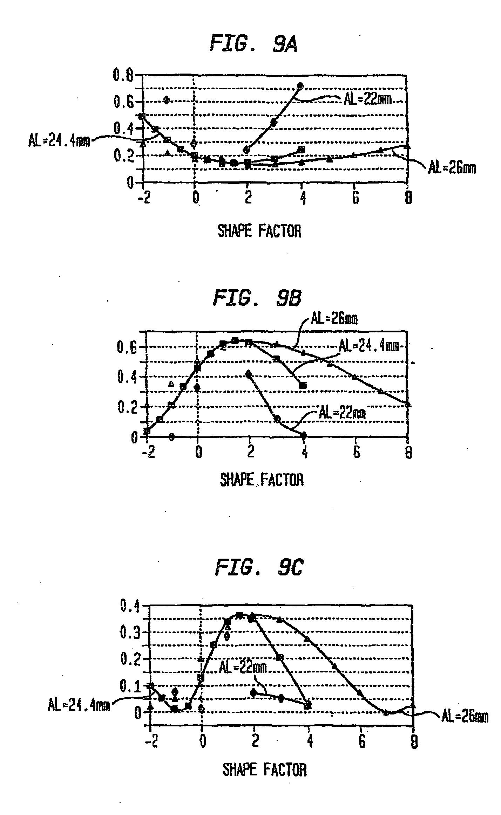

Similar to corneal radius, it was discovered that an optimal shape factor for an IOL can vary as a function of the eye's axial length. By way of example, Table 7 below presents the results of simulations for optical performance of a plurality of IOLs having shape factors in a range of -2 to 8 for a plurality of different axial lengths (ALs). The model eye utilized for these simulations was characterized by an ACD = 4.9 mm, a corneal radius (r) = 7.72 mm, and a corneal asphericity (Q) = -0.26. The graphical representation of these simulations are provided in FIGURE 9A, 9B and 9C for spherical aberration, MTF at 50 lp/mm and MTF at 100 lp/mm, respectively.

**Table 7**

| X | SA (micron) | | | MTF@50lp/mm | | | MTF@100lp/mm | | |
|---|---|---|---|---|---|---|---|---|---|
| | AL=22.0 | AL=24.4 | AL=26.0 | AL=22.0 | AL=24.4 | AL=26.0 | AL=22.0 | AL=24.4 | AL=26.0 |
| | mm | mm | mm | mm | mm | mm | mm | mm | mm |
| -2 | - | 0.478 | 0.285 | - | 0.037 | 0.209 | - | 0.095 | 0.021 |
| -1.5 | - | 0.386 | - | - | 0.117 | - | - | 0.051 | - |
| -1 | 0.609 | 0.307 | 0.215 | 0.000 | 0.212 | 0.364 | 0.078 | 0.011 | 0.047 |
| -0.5 | - | 0.244 | - | - | 0.331 | - | - | 0.016 | - |
| 0 | 0.281 | 0.195 | 0.166 | 0.322 | 0.455 | 0.507 | 0.015 | 0.128 | 0.200 |
| 0.5 | - | 0.162 | - | - | 0.555 | - | - | 0.250 | - |
| 1 | 0.168 | 0.142 | 0.138 | 0.591 | 0.615 | 0.596 | 0.284 | 0.334 | 0.318 |
| 1.5 | - | 0.134 | - | - | 0.637 | - | - | 0.366 | - |
| 2 | 0.240 | 0.138 | 0.127 | 0.407 | 0.625 | 0.629 | 0.070 | 0.348 | - |
| 3 | 0.441 | 0.174 | 0.132 | 0.122 | 0.516 | 0.616 | 0.054 | 0.199 | 0.345 |
| 4 | 0.718 | 0.239 | 0.147 | 0.011 | 0.340 | 0.565 | 0.030 | 0.021 | 0.275 |
| 5 | - | - | 0.171 | - | - | 0.488 | - | - | 0.176 |
| 6 | - | - | 0.202 | - | - | 0.395 | - | - | 0.075 |
| 7 | - | - | 0.237 | - | - | 0.302 | - | - | 0.001 |
| 8 | - | - | 0.274 | - | - | 0.222 | - | - | 0.024 |

The above simulations indicate that while for a long axial length (e.g., an axial length of about 26 mm), IOLs having shape factors over a wide range (e.g., in a range of about -1 to about 8) provide substantially similar performance, for a short axial length (e.g., an axial length of about 22 mm), an optimal IOL shape factor lies in a range of about 0 to about 2 (preferably in a range of about 0.5 to about 2). Further, the peak of optical performance exhibits a shift as a function of axial length variation.

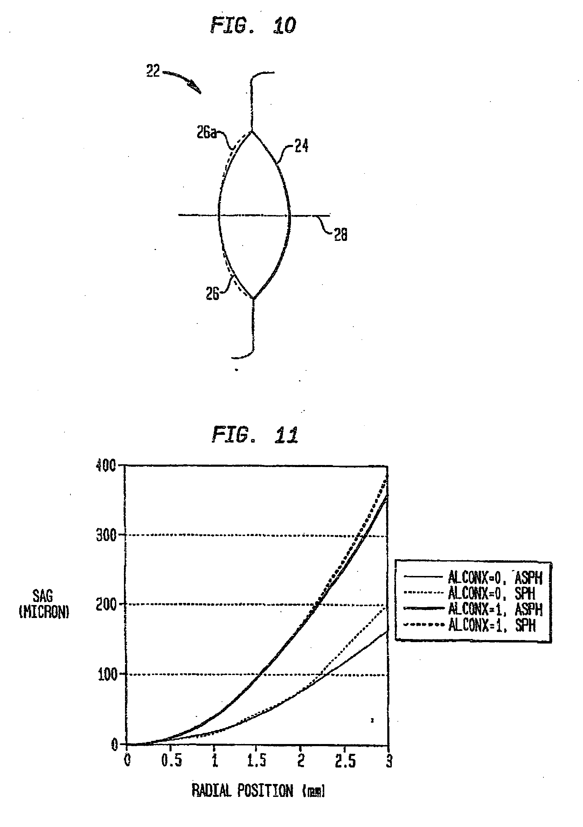

An anterior or a posterior surface of the IOL includes an aspherical base profile selected to compensate for the corneal spherical aberration. Alternatively, both anterior and posterior surfaces can be aspherical so as to collectively provide a selected degree of compensation for the corneal spherical aberration. By way of example, FIGURE 10 shows an IOL 22 according to one embodiment of the invention that includes an optic having a spherical posterior surface 24 and an aspherical anterior surface 26. More specifically, the anterior surface 26 is characterized by a base profile that is substantially coincident with a putative spherical profile 26a (shown by dashed lines) for small radial distances from an optical axis 28 but deviates from that spherical profile as the radial distance from the optical axis increases. In this embodiment, the aspherical anterior surface can be characterized by the following relation:

$$z = \frac{cr^2}{1+\sqrt{1-(1+k)c^2r^2}} \qquad \text{Eq. (2)}$$

wherein,

$c$ denotes the curvature of the surface at its apex (at its intersection with the optical axis),
$r$ denotes the radial distance from the optical axis, and
$k$ denotes the conic constant.

In some embodiments, the conic constant k can range from about -1162 to about -19 (e.g., from about -73 to about -27) and the shape factor of the lens can range from about -0.5 to about 4, and more preferably, from about 0 to about 2. To show the efficacy of such aspherical IOLs in reducing the corneal spherical aberrations, two aspherical IOLs were theoretically designed. The IOLs were assumed to be formed of an acrylic polymer commonly known as Acrysof.

One of the IOLs was selected to have a shape factor of zero (X = 0) while the other was chosen to have a shape factor of 1 (X =1). The edge thickness for each IOL was fixed at 0.21 mm For the IOL with X = 0, the anterior and posterior radii were set, respectively, at 22.934 mm and -22.934 mm, the central thickness was set at 0.577 mm and the anterior surface asphericity (i.e., the conic constant) was selected to be -43.656. For the IOL with X =1, the posterior surface was selected to be flat while the radius of the anterior surface was set at 11.785 mm The central thickness of this lens was 0.577 mm and the anterior surface was assumed to have an asphericity characterized by a conic constant of -3.594. FIGURE 11 shows the sag of the anterior surfaces of these exemplary IOLs as a function of radial distance from the optical axis.

The simulations of the optical performances of these two IOL designs in the aforementioned eye model show a reduction of the total RMS wavefront errors to about 0.000841 waves in case of the IOL having a shape factor that approaches zero and to about 0.000046 in case of the IOL having a shape factor of unity.

Another factor that can affect the optical performance of an IOL is its effective position. The effective lens position (e.g., defined here as the location of the principal plane relative to the posterior surface) can vary as a function of the lens's shape. The location of the second principal plane ($PP_2$) relative to the apex of the posterior surface can be defined by the following relation:

$$PP_2 = \frac{-n_1 dF_1}{n_2 F_L} \qquad \text{Eq. (3)}$$

wherein $n_1$ and $n_2$ denote, respectively, the refractive indices of the IOL and the surrounding medium, $F_1$ represents the optical power of the anterior surface and $F_2$ represents the optical power of the lens, and $d$ is the lens's central thickness. The haptics plane (the anchor plane for the implanted IOL) located at the central-line of the lens edge can have a distance from the apex of the posterior surface specified as:

$$HL = Sag_2 + \frac{ET}{2} \qquad \text{Eq. (4)}$$

wherein $ET$ denotes the lens's edge thickness and $Sag_2$ denotes the sag height of the posterior surface at the lens's edge. Utilizing the above Equations (3) and (4), the location of the second principal point relative to the haptics plane can be defined as follow:

$$\Delta PP_2 = Sag_2 + \frac{ET}{2} - \frac{n_1 dF_1}{n_2 F_L} \qquad \text{Eq. (5)}$$

wherein $\Delta PP_2$ denotes an offset shift of the principal plane, and the other parameters are defined above.

By way of example, the 2nd principal plane shift for the aforementioned IOL having a shape factor of zero (X = 0) was calculated (by utilizing the above equations) across a power range of 0 to about 35 D as +/- 0.03 mm, while the corresponding shift for the IOL having a shape factor of unity (X =1) was calculated as +/- 0.15 mm.

To better appreciate the enhanced optical performance provided by the IOLs of the invention, some of the major factors contributing to the variability of post-operative refractive errors can be considered. These factors are generally classified into three categories: biometric data errors ($\Delta Biometric$), IOL power errors ($\Delta IOLPower$) and high-order aberration contributions ($\Delta Aberration$). An overall variability ($Rx$) can be calculated based on these factors by utilizing, e.g., the following relation:

$$RxError = \sqrt{\Delta Biometric^2 + \Delta IOLPower^2 + \Delta Aberration^2} \qquad \text{Eq. (6)}$$

The Δ*Biometric* can, in turn, be defined in accordance with the following relation:

$$\Delta Biometric = \sqrt{\Delta k^2 + \Delta AL^2 + \Delta ACD^2} \qquad \text{Eq. (7)}$$

wherein Δ*k* denotes the error in keratometric measurement, Δ*AL* denotes the error in axial length measurement, and Δ*ACD* denotes the error in the anterior chamber depth measurement. The Δ*IOLPower* can be defined in accordance with the following relation:

$$\Delta IOLPower = \sqrt{\Delta IOLStep^2 + \Delta IOLTol^2 + \Delta ELP^2} \qquad \text{Eq. (8)}$$

wherein Δ*IOLStep* denotes the variability caused by the use of IOLs whose optical powers differ by finite steps for correcting patients' refractive errors that vary over a continuous range, Δ*IOLTol* denotes manufacturing power tolerance, and Δ*ELP* denotes the variability in the shift of the IOL effective position across the power range. Further, Δ*Aberration* can be defined in accordance with the following relation:

$$\Delta Aberration = \sqrt{\Delta Astig^2 + \Delta SA^2 + \Delta Other^2} \qquad \text{Eq. (9)}$$

wherein Δ*Astig*, Δ*SA*, Δ*Other* denote, respectively, astigmatic, spherical and other higher order aberrations.

The optical performance of the aforementioned exemplary IOL designs having shape factors (X) of zero and unity were evaluated based on estimated *Rx* variability for three conditions: (I) uncorrected visual acuity (i.e., in the absence of corrective spectacles) with IOL power step of 0.5 D (UCVA), (2) uncorrected visual acuity with a refined IOL power step of 0.25 D (UCVA+) and (3) best corrected visual acuity (i.e., utilizing optimal corrective spectacles) (BCVA). The variability due to biometric measurements was estimated from information available in the literature. The focus of the analysis relates to estimating contributions of the spherical aberration, errors due to IOL misalignments, and the 2nd principal plane (PPL) shifts. For comparison purposes, a baseline value of 0.65 D was assumed for UCVA and UCVA+ and a baseline value of 0.33 D was assumed for BCVA, for eyes with spherical IOLs. Table 8 below lists absolute and percentage reductions in Rx relative to the baseline values for the two IOLs:

**Table 8**

|  | IOL with X = 0 | | IOL with X = 1 | |
|---|---|---|---|---|
| UCVA | -0.03 D | -4.39% | 0.00 D | 0.45% |
| UCVA+ | -0.05 D | -7.13% | -0.01 D | -2.16% |
| BCVA | -0.03 D | -8.53% | -0.05 D | -13.87% |

The information presented in Table 8 shows that reductions in *Rx* variability are achieved for both IOLs (X = 0, and X =1), thus indicating improved optical performance of those lenses. For the IOL with a vanishing shape factor (X = 0), the visual benefits are almost evenly distributed among UCVA, UCVA+ and BCVA while for the other IOL (X=1), the visual benefit associated with BCVA is more pronounced.

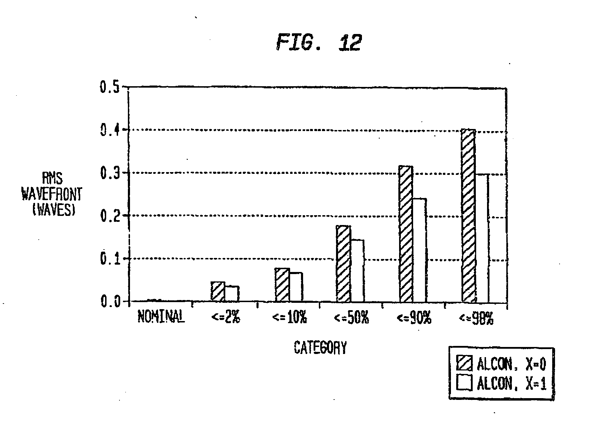

A variety of known manufacturing techniques can be employed to fabricate the lenses of the invention. The manufacturing tolerances can also affect the optical performance of an IOL. By way of example, such tolerances can correspond to variations of, e.g., surface radii, conic constant, surface decentration, surface tilt, and surface irregularity, with tolerances associated with surface asphericity (conic constant) generally playing a more important role that others in affecting optical performance. Simulations, however, indicate that the IOL's misalignments upon implantation in the eye are typically more significant factors in degrading optical performance than manufacturing tolerances (e.g., manufacturing errors can be nearly 10 times less than misalignment errors). By way of further illustration, the optical performance of the aforementioned aspherical lenses with X = 0 and X =1, implanted in the aforementioned eye model, was theoretically investigated by employing Monte Carlo simulations. More specifically, 500 hypothetical lenses were generated under constraints of typical manufacturing tolerances and were randomly oriented relative to the cornea. For example, the tolerances associated with the surface radii, surface irregularities, and surface decentration and tilt were assumed to be, respectively, within +/- 0.1 mm, 2 fringes, 0.05 mm and 0.5 degrees. The results of the Monte Carlo simulations are summarized in FIGURE 12. More than 50% of the simulated eyes exhibit an RMS wavefront error that is less than about 0.2 waves (about 0.08 D equivalent defocus). For the lens having X = 1, about 98% of the simulated eyes show a wavefront error less than about 0.3 waves (about 0.12 D).

Those having ordinary skill in the art will appreciate that various changes can be made to the above embodiments without departing from the scope of the invention.

**Claims**

1.  An ophthalmic lens (10,22) comprising
    an optic (12) having an anterior surface (14,26) and a posterior surface (16,24),
    said optic exhibiting a shape factor, defined as a ratio of the sum of the anterior and posterior curvatures to the difference of such curvatures,
    and said optic being formed from a polymeric biocompatible material, **characterized in that**;
    at least one of the anterior surface and the posterior surface has an aspherical base profile (26), wherein said aspherical base profile is **characterized by** a conic constant (Q) in a range of - 73 to - 27, and
    wherein the shape factor is in a range of- 0.5 to 4.

2.  The ophthalmic lens of claim 1, wherein said optic (12) exhibits a shape factor in a range of 0 to 2.

3.  The ophthalmic lens of claim 1, wherein both of said surfaces (14,16; 24,26) have a generally convex profile.

4.  The ophthalmic lens of claim 1, wherein one of said surfaces (14,16; 24,26) has a generally convex profile and the other surface has a substantially flat profile.

5.  The ophthalmic lens of claim 1, wherein one of said surfaces (14,16; 24,26) has a generally concave profile and the other surface has a substantially flat profile.

6.  The ophthalmic lens of claim 1, wherein one of said surfaces (14,16; 24,26) has a generally concave profile and the other surface has generally convex profile.

7.  The ophthalmic lens of claim 1, wherein said aspherical base profile (26) is defined by the following relation:

$$z = \frac{cr^2}{1+\sqrt{1-(1+k)c^2r^2}}$$

wherein,

   $c$ denotes the curvature of the surface at its apex (at its intersection with the optical axis (18,28) of the lens),
   $r$ denotes the radial distance from the optical axis, and
   $k$ denotes the conic constant,

wherein

   $c$ is in the range of 0.0152 mm$^{-1}$ to 0.0659 mm$^{-1}$,
   $r$ is in a range of 0 to 5 mm, and
   $k$ is in a range of - 73 to - 27.

8.  The ophthalmic lens of claim 1, wherein said surfaces (24,26) cooperatively provide a refractive optical power in a range of 16D to 25D.

9. The ophthalmic lens of claim 1, wherein said polymeric material is selected from the group consisting of acrylic, silicone and hydrogel materials.

10. The ophthalmic lens of any of claims 1 to 9, wherein said lens (10,22) comprises an intraocular lens.

11. The intraocular lens of claim 10,
having haptics (20) sized to fit within an eye having a corneal radius equal to or less than about 7.1 mm, and said optic exhibiting a shape factor in a range of - 0.5 to 4, preferably in a range of + 0.5 to 4, most preferably in a range of 1 to 3,
or having haptics (20) sized to fit within an eye having a corneal radius in a range of 7.1 mm to 8.6 mm, and said optic exhibiting a shape factor in a range of 0 to 3, preferably in a range of + 0.5 to 3, most preferably in a range of 1 to 2,
or having haptics (20) sized to fit within an eye having a corneal radius equal to or greater than about 8.6 mm, and said optic exhibiting a shape factor in a range of + 0.5 to 2, preferably in a range of 1 to 2.

12. The intraocular lens of claim 10, having an axial length equal to or less than about 22 mm, and said optic (12) exhibiting a shape factor in a range of 0 to 2, preferably in a range of 0.5 to 2.

**Patentansprüche**

1. Ophthalmische Linse (10, 22), mit:

   einer Optik (12) mit einer anterioren Oberfläche (14, 26) und einer posterioren Oberfläche (16, 24),
   wobei die Optik einen Formfaktor hat, der als Verhältnis der Summe der anterioren und posterioren Krümmungen zur Differenz dieser Krümmungen definiert ist,
   und wobei die Optik aus einem biokompatiblen Polymermaterial gebildet ist,
   **dadurch gekennzeichnet, dass**
   mindestens eine der anterioren und posterioren Oberfläche ein asphärisches Basisprofil (26) hat, wobei das asphärische Basisprofil durch eine konische Konstante (Q) im Bereich von -73 bis -27 **gekennzeichnet** ist, und und wobei der Formfaktor im Bereich zwischen -0,5 bis 4 liegt.

2. Ophthalmische Linse nach Anspruch 1, bei der die Optik (12) einen Formfaktor im Bereich von 0 bis 2 aufweist.

3. Ophthalmische Linse nach Anspruch 1, bei der die beiden Oberflächen (14, 16; 24, 26) ein allgemein konvexes Profil haben.

4. Ophthalmische Linse nach Anspruch 1, bei der eine der Oberflächen (14, 16; 24, 26) ein allgemein konvexes Profil und die andere Oberfläche ein im Wesentlichen ebenes Profil hat.

5. Ophthalmische Linse nach Anspruch 1, bei der eine der Oberflächen (14, 16; 24, 26) ein allgemein konkaves Profil und die andere Oberfläche ein im Wesentlichen ebenes Profil hat.

6. Ophthalmische Linse nach Anspruch 1, bei der eine der Oberflächen (14, 16; 24, 26) ein allgemein konkaves Profil und die andere Oberfläche ein allgemein konvexes Profil hat.

7. Ophthalmische Linse nach Anspruch 1, bei der das asphärische Basisprofil (26) durch die folgende Beziehung definiert ist:

$$z = \frac{cr^2}{1 + \sqrt{1 - (1+k)c^2 r^2}}$$

dabei bedeuten:

c Krümmung einer Oberfläche in ihrem Scheitelpunkt (im Schnittpunkt mit der optischen Achse (18, 18) der Linse),
r radialer Abstand von der optischen Achse,
k konische Konstante,

wobei

c im Bereich von 0,0152 mm$^{-1}$ bis 0,0659 mm$^{-1}$ liegt,
r im Bereich von 0 bis 5 mm liegt, und
k im Bereich von -73 bis -27 liegt.

8. Ophthalmische Linse nach Anspruch 1, bei der die Oberflächen (24, 26) gemeinsam eine optische Brechkraft im Bereich von 16D bis 25D bereitstellen.

9. Ophthalmische Linse nach Anspruch 1, bei der das Polymermaterial aus einer Gruppe bestehend aus Acryl-, Silicon- und Hydrogel-Materialien gewählt wird.

10. Ophthalmische Linse nach einem der Ansprüche 1 bis 9, bei der die Linse (10, 22) eine Intraokularlinse aufweist.

11. Intraokularlinse nach Anspruch 10, mit Haptiken (20), die so dimensioniert sind, dass sie in ein Auge mit einem Hornhautradius gleich oder kleiner als ca. 7,1 mm passen, und bei der die Optik einen Formfaktor im Bereich von -0,5 bis 4, vorzugsweise im Bereich von +0,5 bis 4 und am meisten bevorzugt im Bereich von 1 bis 3 aufweist, oder mit Haptiken (20), die so dimensioniert sind, dass sie in ein Auge mit einem Hornhautradius im Bereich von 7,1 mm bis 8,6 mm passen, und bei der die Optik einen Formfaktor im Bereich von 0 bis 3, vorzugsweise im Bereich von +0,5 bis 3 und am meisten bevorzugt im Bereich von 1 bis 2 aufweist, oder mit Haptiken (20), die so dimensioniert sind, dass sie in ein Auge mit einem Hornhautradius gleich oder größer als ca. 8,6 mm passen, und bei der die Optik einen Formfaktor im Bereich von +0,5 bis 2, vorzugsweise im Bereich von 1 bis 2 aufweist.

12. Intraokularlinse nach Anspruch 10, mit einer axialen Länge gleich oder kleiner ca. 22 mm und bei der die Optik (12) einen Formfaktor im Bereich von 0 bis 2, vorzugsweise im Bereich von 0,5 bis 2 aufweist.

**Revendications**

1. Lentille ophtalmique (10, 22) comprenant
une optique (12) ayant une surface antérieure (14, 26) et une surface postérieure (16, 24),
ladite optique présentant un facteur de forme, défini comme étant le rapport de la somme des courbures antérieure et postérieure sur la différence de ces courbures,
et ladite optique étant formée d'un matériau biocompatible polymère,
**caractérisée en ce que** ;
au moins une parmi la surface antérieure et la surface postérieure a un profil de base (26) asphérique, ledit profil de base asphérique étant **caractérisé par** une constante conique (Q) dans une plage de -73 à -27, et le facteur de forme étant dans une plage de -0,5 à 4.

2. Lentille ophtalmique selon la revendication 1, dans laquelle ladite optique (12) présente un facteur de forme dans une plage de 0 à 2.

3. Lentille ophtalmique selon la revendication 1, dans laquelle les deux surfaces (14, 16 ; 24, 26) ont un profil principalement convexe.

4. Lentille ophtalmique selon la revendication 1, dans laquelle une desdites surfaces (14, 16 ; 24, 26) a un profil principalement convexe et l'autre surface a un profil essentiellement plat.

5. Lentille ophtalmique selon la revendication 1, dans laquelle une desdites surfaces (14, 16 ; 24, 26) a un profil principalement concave et l'autre surface a un profil essentiellement plat.

6. Système selon la revendication 1, dans laquelle une desdites surfaces (14, 16 ; 24, 26) a un profil principalement concave et l'autre surface a un profil principalement convexe.

7. Lentille ophtalmique selon la revendication 1, dans laquelle ledit profil de base (26) asphérique est défini par la relation suivante :

$$z = \frac{cr^2}{1 + \sqrt{1 - (1+k)c^2r^2}}$$

dans laquelle,

$c$ indique la courbure de la surface au niveau de son sommet (au niveau de son intersection avec l'axe optique (18, 28) de la lentille),
$r$ indique la distance radiale à partir de l'axe optique, et
$k$ indique la constante conique,
$c$ étant dans la plage de 0,0152 mm$^{-1}$ à 0,0659 mm$^{-1}$,
$r$ étant dans une plage de 0 à 5 mm, et
$k$ étant dans une plage de -73 à -27.

8. Lentille ophtalmique selon la revendication 1, dans laquelle lesdites surfaces (24, 26) fournissent en commun une puissance de réfraction optique dans une plage de 16D à 25D.

9. Lentille ophtalmique selon la revendication 1, dans laquelle ledit matériau polymère est sélectionné parmi le groupe constitué des matériaux acrylique, silicone et hydrogel.

10. Lentille ophtalmique selon l'une quelconque des revendications 1 à 9, dans laquelle ladite lentille (10, 22) comprend une lentille intraoculaire.

11. Lentille intraoculaire selon la revendication 10,
ayant une haptique (20) dimensionnée pour tenir à l'intérieur d'un oeil ayant un rayon cornéen inférieur ou égal à environ 7,1 mm, et ladite optique présentant un facteur de forme dans une plage de -0,5 à 4, de manière préférée dans une plage de +0,5 à 4, de manière plus préférée dans une plage de 1 à 3,
ou ayant une haptique (20) dimensionnée pour entrer à l'intérieur d'un oeil ayant un rayon cornéen dans une plage de 7,1 mm à 8,6 mm, et ladite optique présentant un facteur de forme dans une plage de 0 à 3, de manière préférée dans une plage de +0,5 à 3, de manière plus préférée dans une plage de 1 à 2,
ou ayant une haptique (20) dimensionnée pour entrer à l'intérieur d'un oeil ayant un rayon cornéen supérieur ou égal à environ 8,6 mm, et ladite optique présentant un facteur de forme dans une plage de +0,5 à 2, de manière préférée dans une plage de 1 à 2.

12. Lentille intraoculaire selon la revendication 10, ayant une longueur axiale inférieure ou égale à environ 22 mm, et ladite optique (12) présentant un facteur de forme dans une plage de 0 à 2, de manière préférée dans une plage de 0,5 à 2.

## FIG. 1

## FIG. 2

## FIG. 3

## FIG. 4A

## FIG. 4B

## FIG. 5A

SHAPE FACTOR X

MTF @50 lp/mm
MTF @100 lp/mm

## FIG. 5B

SHAPE FACTOR X

SA (WAVES)

## FIG. 6A

SHAPE FACTOR X

SA (WAVES)

## FIG. 6B

SHAPE FACTOR X

MTF @50 lp/mm
MTF @100 lp/mm

## FIG. 7A

SHAPE FACTOR

## FIG. 7B

SHAPE FACTOR

## FIG. 7C

SHAPE FACTOR

## FIG. 8A

## FIG. 8B

## FIG. 8C

## FIG. 9A

SHAPE FACTOR

## FIG. 9B

SHAPE FACTOR

## FIG. 9C

SHAPE FACTOR

## FIG. 10

## FIG. 11

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4126144 A
- US 5050981 A, Roffman, Jeffrey H.
- US 2003093149 A1, Alan N. Glazier

**Non-patent literature cited in the description**

- *Patent Abstracts of Japan,* 18 August 1992, vol. 16 (387